# DEMANDE DE BREVET EUROPEEN

(11) **EP 3 659 652 A1**
(43) Date de publication de la demande: **03.06.2020**
(21) Numéro de dépôt: 19211944.4
(22) Date de dépôt: 27.11.2019
(51) Int. Cl.: A61M 5/32

(54) **ORGANE DE RETRAIT D'UN CAPUCHON DE PROTECTION D'AIGUILLE**

(30) Priorité: 27.11.2018 FR 1871946
(71) Demandeur: NEMERA LA VERPILLIERE, 38290 La Verpillière (FR)
(72) Inventeur: DUMET, Clément, 69120 Vaulx en Velin (FR); TODESCO, Marc, 38230 Tignieu-Jameyzieu (FR); DUGAND, Pascal, 38780 Estrablin (FR); CAMBA, José, 01500 Amberieu en Bugey (FR); ABOT, Guillaume, 22560 Trébeurden (FR)
(74) Mandataire: LLR

(57) **Abrégé**

L'invention concerne un organe de retrait (217) d'un capuchon (3) de protection d'aiguille, pour un dispositif d'injection (5) comprenant une aiguille (21) et un capuchon (3) amovible de protection d'aiguille (21), l'organe de retrait (217) comprenant un logement (23) présentant un axe longitudinal (L) et étant apte à recevoir le capuchon (3) selon un sens d'insertion, le logement (23) étant délimité par au moins une première (213) et une seconde zones d'appui (215) destinées à servir d'appui au capuchon (3) logé dans le logement (23), la première zone (213) étant portée par un élément de blocage (1), l'élément de blocage (1) étant mobile par rapport au logement (23) entre :
- une configuration d'insertion du capuchon (3), dans laquelle les projections orthogonales de la première zone (213) et de la seconde zone (215) dans un plan transversal sensiblement perpendiculaire à l'axe longitudinal (L) sont séparées d'une distance d'insertion (Di), et
- une configuration de blocage du capuchon (3), dans laquelle la première zone (213) et la seconde zone (215) sont situées dans des plans transversaux distincts, et dans laquelle les projections orthogonales de la première zone (213) et de la seconde zone (215) dans ledit plan transversal sont séparées d'une distance de blocage (Db), inférieure à la distance d'insertion (Di) et apte à bloquer la translation du capuchon (3) par rapport à l'organe de retrait (217) dans un sens de retrait opposé au sens d'insertion.

## Description

L'invention concerne un organe de retrait d'un capuchon de protection d'aiguille, un dispositif d'injection comprenant un tel organe de retrait, par exemple une seringue d'injection, comprenant éventuellement un dispositif de sécurité ou un dispositif auto-injecteur. Elle concerne également un procédé d'assemblage d'un organe de retrait sur un dispositif d'injection. L'organe de retrait est capable de se solidariser au capuchon de protection, de sorte que le retrait de cet organe entraîne le retrait du capuchon de protection.

L'invention concerne plus particulièrement le retrait, avant l'injection, du capuchon protégeant l'aiguille. La surface de préhension du capuchon peut être de taille relativement réduite et de ce fait, le capuchon peut être difficile à retirer, en particulier pour les personnes souffrant de polyarthrite. Il a déjà été proposé, dans la demande FR3011186, un organe de retrait du capuchon de protection d'aiguille. Cet organe de retrait comporte des éléments d'accrochage du capuchon qui s'agrippent à l'arrière de celui-ci. Pour être utilisé, cet organe de retrait requiert donc que le dispositif d'injection et le capuchon soient assemblés de manière à ménager, à l'arrière du capuchon, un espace permettant un accrochage efficace de l'organe de retrait. Par conséquent cet organe de retrait est utilisable uniquement sur des dispositifs d'injection présentant une configuration adaptée.

L'invention a notamment pour but de fournir un organe de retrait utilisable avec davantage de dispositifs d'injection.

A cet effet, l'invention a notamment pour objet un organe de retrait d'un capuchon de protection d'aiguille, pour un dispositif d'injection comprenant une aiguille et un capuchon amovible de protection d'aiguille, l'organe de retrait comprenant un logement présentant un axe longitudinal et étant apte à recevoir le capuchon selon un sens d'insertion, le logement étant délimité par au moins une première et une seconde zones d'appui dites première et seconde zone destinées à servir d'appui au capuchon logé dans le logement, la première zone étant portée par un élément de blocage mobile par rapport au logement entre :
- au moins une configuration d'insertion du capuchon, dans laquelle des projections orthogonales de la première zone et de la seconde zone dans un plan transversal sensiblement perpendiculaire à l'axe longitudinal sont séparées d'une distance d'insertion, et ;
- une configuration de blocage du capuchon, dans laquelle la première zone et la seconde zone sont situées dans des plans transversaux distincts, et dans laquelle les projections orthogonales de la première zone et de la seconde zone dans ledit plan transversal sont séparées d'une distance de blocage inférieure à la distance d'insertion et apte à bloquer la translation du capuchon par rapport à l'organe de retrait dans un sens de retrait opposé au sens d'insertion.

On entend par « plan transversal » un plan non parallèle à l'axe longitudinal, de préférence sensiblement perpendiculaire à l'axe longitudinal.

Ainsi, du fait que la distance d'insertion est supérieure à la distance de blocage, le passage présenté au capuchon lors de son insertion dans le sens d'insertion est plus large que le passage présenté au même capuchon en configuration de blocage. En effet, grâce à la différence de distance, la première et la seconde zones délimitent, en configuration d'insertion, un passage plus grand pour le capuchon qui est inséré dans le sens d'insertion, du fait que dans ce sens d'insertion, ce passage est délimité par la projection orthogonale de la première et de la seconde zones sur le plan transversal. En d'autres termes, on propose d'assurer le blocage du capuchon dans l'organe de retrait par un arc-boutement de l'élément de blocage. Ainsi, le blocage du capuchon dans l'organe de retrait ne nécessite pas d'accrocher le capuchon à l'arrière de celui-ci ; il ne nécessite d'ailleurs pas la présence d'un épaulement sur le capuchon. L'organe de retrait proposé peut donc s'adapter à tout type de dispositif d'injection, quelle que soit sa forme, et en particulier quelle que soit la forme de son capuchon.

En outre, le blocage est assuré par des frottements du capuchon par au moins la première et la seconde zones, ce qui est plus fiable qu'un blocage assuré par des pattes élastiques, susceptibles de se décrocher accidentellement au moment où l'on retire le capuchon et qui peuvent requérir des moyens supplémentaires de solidarisation au capuchon.

De préférence, les première et seconde zones sont disposées, en configuration d'insertion, dans un même plan longitudinal passant par l'axe longitudinal, ou dans des plans longitudinaux distincts. En configuration de blocage, les première et seconde zones sont dans des plans transversaux distincts davantage éloignés l'un de l'autre qu'en position d'insertion. On comprend par ailleurs que, plus les première et seconde zones d'appui sont situées dans des plans transversaux distincts, plus la distance d'appui séparant leurs projections orthogonales dans un plan transversal sensiblement perpendiculaire à l'axe longitudinal est réduite. La réduction de cette distance d'appui permet, par arc-boutement, de bloquer le capuchon dans l'organe de retrait.

On comprend que le logement pour le capuchon correspond à un espace laissé libre pour que le capuchon puisse être reçu dans l'organe de retrait pour la protection de l'aiguille en position de stockage du dispositif d'injection, avant son utilisation. Le contour du logement peut avoir une forme quelconque, en passant par les première et seconde zones. Le contour peut être fermé ou ouvert. Il peut avoir une forme géométrique générale telle qu'un cylindre de section circulaire ou une forme géométrique quelconque. Le contour peut en outre présenter des évidements ou encore des saillies internes, notamment pour former des zones d'appui du capuchon.

On comprend par ailleurs que l'élément de blocage peut être rapporté sur le reste de l'organe de retrait ou venu de matière.

On comprend également que le dispositif d'injection peut comprendre une seringue d'injection seule, comprenant un corps pourvu d'une aiguille creuse et destiné à administrer un produit par injection. La seringue d'injection peut prendre éventuellement la forme d'une cartouche sur laquelle est rapportée une aiguille. Le dispositif d'injection peut également comprendre un dispositif de sécurité pour seringue d'injection, ou encore un dispositif auto-injecteur, avec ou sans la seringue d'injection.

L'organe de retrait peut en outre comporter l'une ou plusieurs des caractéristiques suivantes, prises seules ou en combinaison.
- Le logement est délimité en outre par une troisième zone d'appui dite troisième zone, destinée à servir d'appui au capuchon pour empêcher son pivotement par rapport à l'une au moins desdites première et seconde zones dans un plan longitudinal, au moins lors du passage de l'élément de blocage entre ses configurations d'insertion et de blocage. Les première, seconde et troisième zones d'appui sont disposées en quinconce de part et d'autre de l'axe longitudinal et dans au moins deux plans transversaux distincts lorsque l'élément de blocage est en configuration de blocage.

On entend de préférence par « les première, seconde et troisième zones d'appui étant disposées en quinconce de part et d'autre de l'axe longitudinal dans des plans transversaux distincts » le fait que les projections orthogonales de deux des première, seconde et troisième zones d'appui dans un plan transversal sensiblement perpendiculaire à l'axe longitudinal sont disposées d'un côté de l'axe longitudinal alors que la projection orthogonale de l'autre desdites première, seconde et troisième zones d'appui est située de l'autre côté de l'axe longitudinal. La première, seconde et troisième zones d'appui dont la projection orthogonale est située de l'autre côté de l'axe longitudinal (par rapport aux projections orthogonales des deux première, seconde et troisième zones d'appui situées du même côté de l'axe longitudinal) est située axialement entre les deux première, seconde et troisième zones d'appui situées du même côté de l'axe longitudinal. Les côtés de l'axe longitudinal dans lesquels, pour l'un les projections orthogonales de deux desdites première, seconde et troisième zones d'appui se trouvent et, pour l'autre la projection orthogonale de l'autre desdites première, seconde et troisième zones d'appui se trouve, sont séparés par un plan longitudinal passant par l'axe longitudinal. De préférence, les première, seconde et troisième zones d'appui sont disposées dans un même plan longitudinal passant par l'axe longitudinal, en quinconce de part et d'autre de l'axe longitudinal.

On comprend que la troisième zone est portée ici par l'organe de retrait et est en interaction avec le capuchon. De façon alternative, on pourrait envisager d'empêcher le pivotement du capuchon par d'autres moyens, par exemple en prévoyant une troisième zone portée par l'organe de retrait et en interaction avec le dispositif d'injection pour éviter le pivotement du capuchon par rapport à l'organe de retrait.
- L'une au moins des première, seconde et troisième zones forme un moyen de guidage axial du capuchon entre la configuration d'insertion et la configuration de blocage, par exemple une rainure tubulaire disposée dans un fond de l'organe de retrait, ou un bossage faisant saillie d'une paroi tubulaire intérieure de l'organe de retrait, ou encore une seconde ouverture de passage du capuchon. Ce moyen de guidage axial permet d'éviter le pivotement du capuchon par rapport aux première et seconde zones lors de l'insertion du capuchon dans le logement.
- L'élément de blocage comprend une plaque délimitant au moins une première ouverture de passage du capuchon dont le contour définit au moins la première zone, de préférence le contour de la première ouverture de passage définit également l'une au moins des seconde zone et troisième zone. De préférence, la première ouverture définit la deuxième zone, sensiblement diamétralement opposée par rapport à la première zone. Par exemple, la plaque peut être simple avec une seule ouverture ou bien pliée, avec éventuellement plusieurs ouvertures. Lorsque la plaque est pliée et comporte deux ouvertures, la deuxième ouverture peut définir la troisième zone. Selon un autre mode de réalisation, la plaque peut également être pliée et ne comporter qu'une ouverture.
Selon ce mode de réalisation, une des zones d'appui sera formée par l'ouverture, les autres zones d'appui seront portées par l'organe de retrait.

On entend de préférence par « plaque » une pièce dont les dimensions générales de longueur et de largeur sont prépondérantes par rapport à l'épaisseur. La plaque peut éventuellement comprendre des reliefs.

Le contour de la première ouverture peut avoir une forme quelconque. Le contour peut être fermé ou ouvert. Il peut avoir une forme géométrique générale telle qu'un cercle, un polygone tel qu'un pentagone régulier, ou encore un polygone étoilé, ou une forme géométrique quelconque. Le contour peut en outre présenter des évidements ou encore des saillies internes, notamment pour que les zones d'appui présentent une certaine souplesse facilitant l'insertion du capuchon dans le logement.
- L'élément de blocage et l'organe de retrait comportent des formes complémentaires ou chacun une surface plane définissant une butée axiale apte à bloquer la translation de l'élément de blocage par rapport à l'organe de retrait dans le sens de retrait du capuchon dans l'organe de retrait.
- L'élément de blocage est mobile par rapport au logement de façon à prendre également une configuration de repos, dans laquelle les projections orthogonales de la première zone et de la seconde zone dans un plan transversal sont séparées d'une distance de repos inférieure à la distance de blocage.

On comprend que l'élément de blocage peut prendre une configuration de repos correspondant à la position de la première et la seconde zones avant insertion du capuchon. Cette configuration de repos est une configuration dans laquelle l'élément de blocage peut être ou ne pas être déformé élastiquement. On notera que la configuration de repos peut éventuellement être confondue avec la configuration d'insertion.
- La première zone et la seconde zone sont portées par l'élément de blocage.
- L'élément de blocage est solidaire du reste de l'organe de retrait par le biais d'une liaison de type pivot élastique tendant à ramener l'élément de blocage dans sa configuration de blocage. Le pivot peut être mobile en translation dans l'organe de retrait.
- L'élément de blocage est une plaque pliée de façon à définir deux portions s'étendant de part et d'autre d'un axe de pivotement des portions l'une par rapport à l'autre. Au moins une des deux portions comprend une ouverture de passage du capuchon, et les deux portions forment un angle entre elles. L'angle est distinct de 180°, de préférence compris entre 30° et 150°. La variation d'angle de l'élément de blocage entre la configuration d'insertion et la configuration de blocage est de préférence comprise entre 15° et 60°. La variation d'angle de l'élément de blocage entre la configuration de repos et la configuration d'insertion est de préférence comprise entre 0° et 30°.

- Chacune des deux portions comprend une ouverture de passage du capuchon, ainsi que des moyens de fixation audit logement, tels que des ergots coopérant avec au moins une rainure prévue dans le logement, et de préférence avec deux rainures prévues dans le logement.
- Chacune des deux portions comprend une ouverture de passage du capuchon.
- La plaque comprend une ouverture de passage s'étendant sur les deux portions.
- La butée axiale est formée par des formes complémentaires de la plaque et de l'organe de retrait telles que par exemple des ergots coopérant avec au moins une rainure non débouchante prévue de part et d'autre du logement dans l'organe de retrait. De préférence les ergots coopèrent avec deux rainures prévues de part et d'autre du logement dans l'organe de retrait.
- L'organe de retrait comprend une jupe externe de préhension de l'organe de retrait par un utilisateur. Cette jupe est de préférence de forme annulaire ou tronconique, présentant une surface de préhension par l'utilisateur, munie de reliefs de préhension ou encore d'une partie évasée.
- L'élément de blocage comprend du métal ou du plastique, préférentiellement du polypropylène.
- L'élément de blocage est déformable élastiquement entre au moins la configuration d'insertion et la configuration de blocage. En d'autres termes, il passe d'une configuration à l'autre par déformation élastique. De préférence, dans le cas où il peut prendre également une configuration de repos, c'est sous l'effet d'une déformation élastique également.
- L'élément de blocage déformable élastiquement peut être monté dans le logement par le biais d'une liaison encastrement, par exemple par encliquetage ou bouterollage.
- L'élément de blocage passe de la configuration d'insertion du capuchon à la configuration de blocage du capuchon sous l'action d'un élément de poussée mobile par rapport au logement, l'élément de poussée étant distinct du capuchon, entre une position non enfoncée dans laquelle l'élément de blocage est dans la configuration d'insertion et une position enfoncée dans laquelle l'élément de blocage est en configuration de blocage du capuchon sous l'action de l'élément de poussée, l'élément de poussée étant pourvu de moyens de verrouillage aptes à l'immobiliser par rapport au logement dans la position enfoncée.
- L'organe de retrait et l'élément de blocage comportent chacun une surface plane définissant une butée axiale, la portion de l'élément de blocage forme une jupe présentant à son extrémité distale une collerette, et présentant le même axe longitudinal que le logement, la surface plane de l'élément de blocage est comprise dans une surface distale de la collerette de l'élément de blocage, et la surface plane de l'organe de retrait fait partie d'une embase d'une extrémité mobile portant l'élément de poussée. Ainsi, les points d'appui de la collerette sur l'organe de retrait sont répartis sur la collerette ce qui améliore le blocage de l'élément de blocage dans l'organe de retrait, fiabilisant ainsi l'organe de retrait.
- L'élément de blocage et l'organe de retrait comportent des formes complémentaires définissant une butée angulaire apte à bloquer la rotation de l'élément de blocage par rapport à l'organe de retrait de sorte que l'orientation angulaire de l'élément de blocage par rapport à l'élément de poussée permette le passage de la configuration d'insertion du capuchon à la configuration de blocage du capuchon sous l'action de l'élément de poussée.
- Le capuchon est un capuchon de type RNS, pour « rigid needle shield », combinant une partie souple pour l'étanchéité et une partie rigide pour la tenue du capuchon. Ce type de capuchon est utilisé en particulier pour une seringue d'injection pré-remplie.

La projection orthogonale de la distance d'appui entre les première et seconde zones est strictement supérieure à un diamètre du capuchon Dc, de préférence 0,5% supérieure, 1% supérieure, 2% supérieure, ou de préférence encore 5% supérieure au diamètre du capuchon Dc. Le diamètre Dc correspond de préférence au diamètre à mi-hauteur du capuchon, il peut également correspondre au diamètre maximal du capuchon ou encore au diamètre à l'extrémité distale du capuchon. On notera que dans la présente description, la direction distale désigne la direction la plus proche de la peau d'un patient au moment d'une injection, et la direction proximale désigne la direction opposée. En d'autres termes, on pourra dire que la direction distale est celle qui va vers l'avant du dispositif d'injection. En particulier, l'extrémité distale d'une pièce correspond à l'extrémité se trouvant du côté de l'aiguille d'injection, et l'extrémité proximale correspond à l'extrémité opposée.
- L'organe de retrait comporte au moins une extrémité plane complémentaire d'au moins une butée plane d'une extrémité distale du dispositif d'injection, l'extrémité plane et la butée plane étant aptes à coopérer ensemble de manière à bloquer en translation l'organe de retrait sur l'extrémité distale du dispositif d'injection. Ainsi, l'organe de retrait est maintenu bloqué axialement sur le dispositif d'injection.
- L'extrémité plane et la butée plane sont positionnées de manière à ce qu'une rotation de l'organe de retrait par rapport au manchon désengage les butées planes des extrémités plane. Ceci permet ainsi de retirer axialement l'organe de retrait de l'extrémité distale du dispositif d'injection, et de contrôler ainsi le retrait de l'organe de retrait.

L'organe de retrait comporte une forme complémentaire d'une forme d'une extrémité distale du dispositif d'injection, les deux formes étant aptes à coopérer ensemble de manière à bloquer en rotation l'organe de retrait (317) sur l'extrémité distale (311) du dispositif d'injection. Grâce à la coopération des deux formes complémentaires, l'organe de retrait est positionné angulairement sur l'extrémité distale, et retenu dans cette position. De ce fait, le retrait de l'organe de retrait, permettant le retrait du capuchon de protection, nécessite une rotation d'un certain angle de l'organe de retrait par rapport à l'extrémité distale du dispositif d'injection, ce qui permet d'exercer un contrôle sur le retrait de l'organe de retrait, et donc du capuchon de protection. Ainsi, lorsque le dispositif d'injection comprend un dispositif auto-injecteur, et en cas de chute de celui-ci, la séparation entre l'organe de retrait et le dispositif auto-injecteur est empêchée, de même que l'activation involontaire du dispositif auto-injecteur, ce qui est particulièrement avantageux ,. Avantageusement, le dispositif d'injection comprend un dispositif auto-injecteur.
- L'organe de retrait comporte des éléments en forme d'arc de cercle destinés à coopérer avec des évidements en forme de trapèze d'un boîtier extérieur d'un auto-injecteur compris dans un dispositif d'injection de manière à permettre une translation par rotation de l'organe de retrait sur le boîtier extérieur. La pente de l'évidement du boîtier extérieur facilite la translation de l'organe de retrait par rotation et la libération de l'élément en forme d'arc de cercle. Ceci facilite donc le retrait de l'organe de retrait du dispositif d'injection, et donc le retrait du capuchon de protection.

L'invention a également pour objet un dispositif d'injection comprenant un organe de retrait tel que présenté ci-avant.

De préférence, le dispositif d'injection comporte un dispositif de sécurité pour protéger l'utilisateur contre une piqûre de l'aiguille après utilisation.

L'invention a également pour objet un procédé d'assemblage d'un organe de retrait sur un dispositif d'injection comprenant une aiguille et un capuchon amovible de protection d'aiguille. L'organe de retrait comprend un logement présentant un axe longitudinal et étant apte à recevoir le capuchon selon un sens d'insertion. Le logement est délimité par au moins une première et une seconde zones d'appui dites première zone et seconde zone, destinées à servir d'appui au capuchon logé dans le logement, la première zone étant portée par un élément de blocage mobile par rapport au logement. Le procédé comprend au moins les étapes suivantes :
- on insère le capuchon au regard du logement, dans la direction d'insertion, l'élément de blocage étant dans une configuration d'insertion du capuchon, dans laquelle la première zone et la seconde zone sont disposées de sorte que leurs projections orthogonales dans un plan transversal sensiblement perpendiculaire à l'axe longitudinal sont séparées d'une distance d'insertion ;
- on stoppe l'insertion du capuchon dans l'organe de retrait,
- l'élément de blocage prend une configuration de blocage du capuchon dans laquelle la première zone et la seconde zone sont situées dans des plans transversaux distincts, et disposées de sorte que leurs projections orthogonales dans ledit plan transversal sont séparées d'une distance de blocage, inférieure à la distance d'insertion et apte à bloquer la translation du capuchon par rapport à l'organe de retrait dans un sens de retrait opposé au sens d'insertion du capuchon.

On peut insérer le capuchon au regard du logement de façon que le capuchon exerce une pression sur l'élément de blocage pour le faire passer d'une configuration de repos dans laquelle les projections orthogonales de la première zone et de la seconde zone dans un plan transversal sont séparées d'une distance de repos inférieure à la distance de blocage vers une configuration d'insertion et augmenter la distance séparant lesdites projections orthogonales, jusqu'à la distance d'insertion.

On peut faire passer l'élément de blocage de la configuration d'insertion du capuchon à la configuration de blocage du capuchon en déplaçant la première zone par rapport à la seconde zone au moyen d'un élément de poussée que l'on déplace par rapport au logement entre une position non enfoncée dans laquelle l'élément de blocage est dans la configuration d'insertion et une position enfoncée dans laquelle l'élément de blocage est en configuration de blocage du capuchon, et on bloque l'élément de poussée par rapport au logement dans la position enfoncée.

L'invention a également pour objet un procédé d'utilisation d'un organe de retrait assemblé selon un procédé d'assemblage tel que décrit ci-avant, au cours duquel un utilisateur appuie sur une extrémité mobile de l'organe de retrait, montée déplaçable par l'utilisateur par rapport à une surface de préhension de l'organe de retrait, entre une position non enfoncée et une position enfoncée dans laquelle l'élément de blocage est en configuration de blocage du capuchon sous l'action d'un élément de poussée.

Plusieurs modes de réalisation de l'invention, donnés à titre d'exemples non limitatifs sont présentés par la suite à l'appui des figures annexées parmi lesquelles :

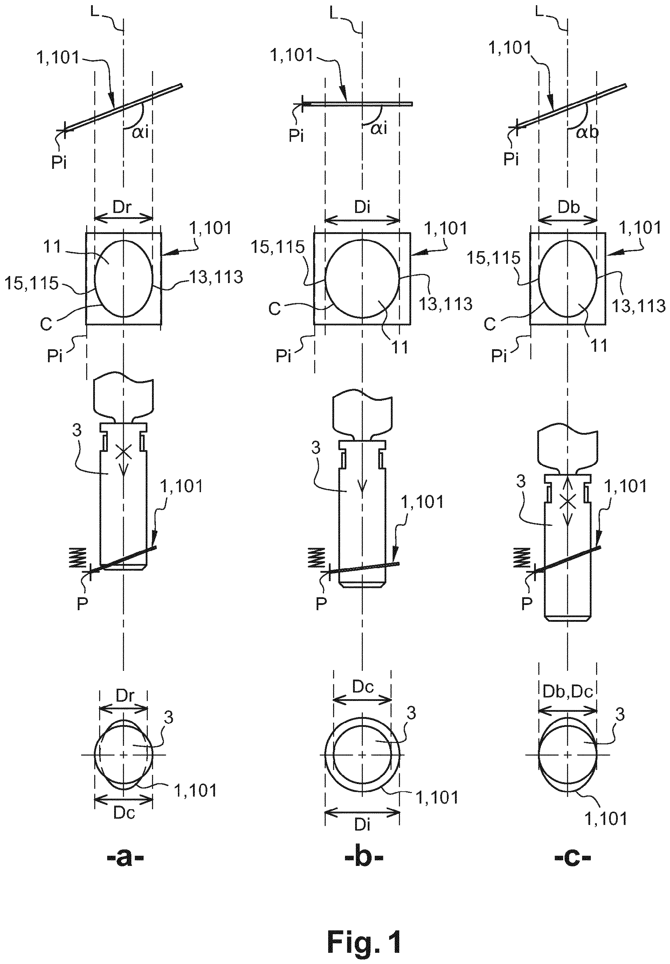

- les figures 1a, 1b, 1c sont des vues schématiques d'un premier principe de fonctionnement de l'invention dans lequel l'élément de blocage est élastiquement déformable entre une configuration de repos (figure 1a), une configuration d'insertion (figure 1b) et une configuration de blocage (figure 1c) ;

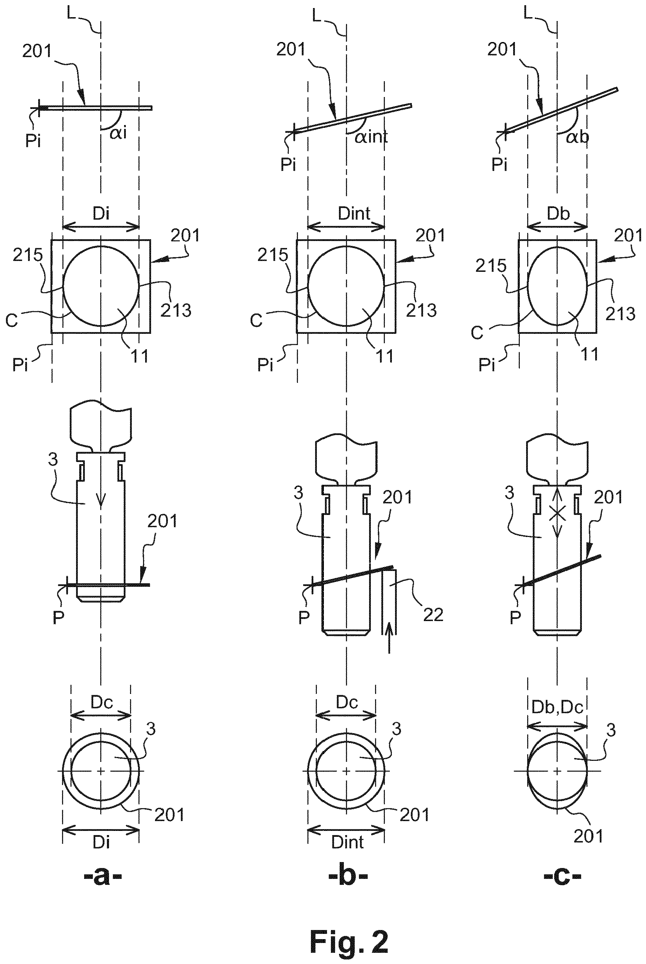

- les figures 2a, 2b, 2c sont des vues schématiques d'un second principe de fonctionnement de l'invention dans lequel l'élément de blocage est déformé au moyen d'un élément de poussée entre une configuration de repos (figure 2a) et une configuration de blocage (figure 2c), en passant par une configuration intermédiaire (figure 2b) ;

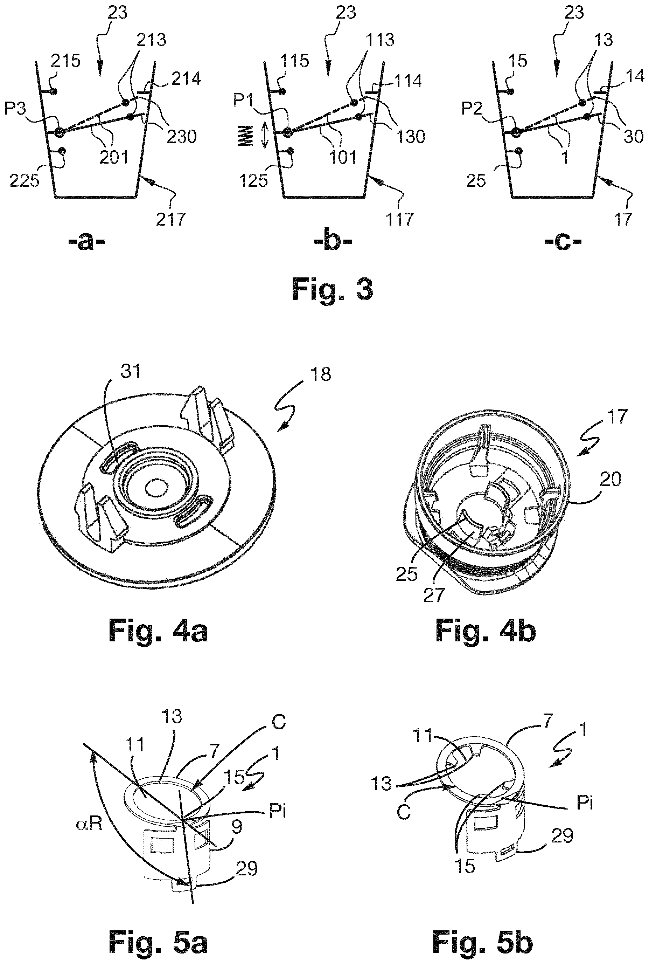

- les figures 3a, 3b, 3c sont des vues schématiques en coupe de côté d'un organe de retrait selon trois modes de liaison entre l'élément de blocage et l'organe de retrait : une liaison pivot élastique (figures 3a), une liaison pivot mobile élastique (figures 3b), une liaison encastrement (figures 3c).

Les figures 1a-c et 2a-c comportent en partie supérieure une vue schématique de côté de l'élément de blocage, en partie médiane supérieure une vue schématique de dessus de l'élément de blocage, en partie médiane inférieure une vue schématique de côté de l'élément de blocage et du capuchon, en partie inférieure une vue schématique du contour du capuchon et du contour de l'ouverture de passage de l'élément de blocage.

Les figures 3a-c illustrent l'élément de blocage en position d'insertion en trait plein et en configuration de blocage en trait mixte fin. La particularité élastique des liaisons et encastrement est symbolisée par un ressort schématisé.

Un premier mode de réalisation du premier principe de fonctionnement de l'invention est illustré par les figures 4a à 7 dans lesquelles :
- les figures 4a et 4b sont des vues en perspective d'une extrémité mobile de l'organe de retrait selon deux variantes de réalisation ;
- les figures 5a et 5b sont des vues en perspective de l'élément de blocage selon deux variantes de réalisation ;
- la figure 6 est une vue en coupe longitudinale de l'ensemble de l'organe de retrait sur le dispositif d'injection, l'élément de blocage de l'organe de retrait étant en configuration d'insertion ;
- la figure 7 est une vue en coupe longitudinale de l'ensemble de l'organe de retrait et du dispositif d'injection, après retrait du capuchon.

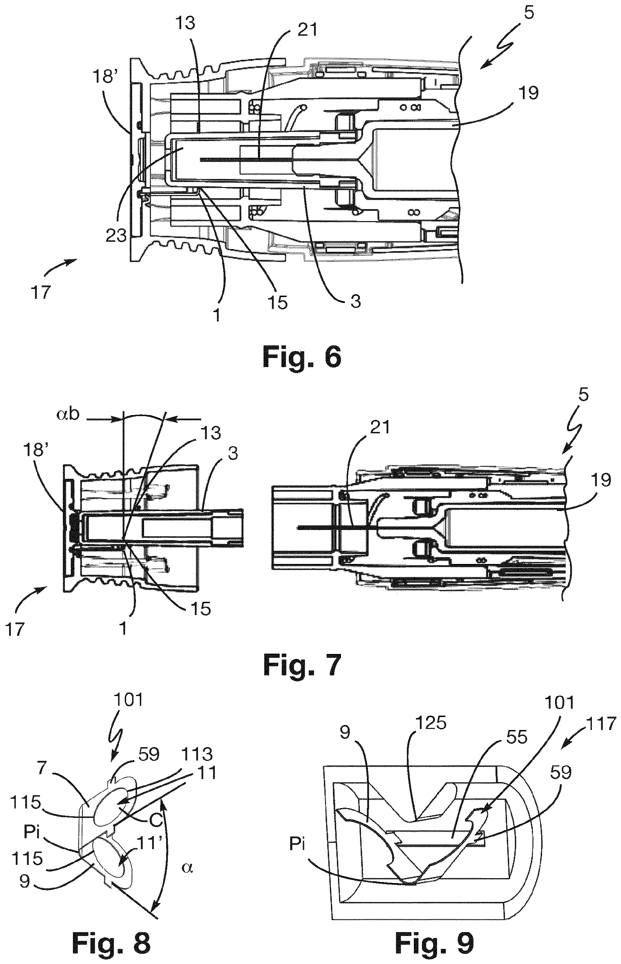

Un second mode de réalisation du premier principe de fonctionnement de l'invention est illustré par les figures 8 à 12d dans lesquelles :
- la figure 8 est une vue en perspective de l'élément de blocage montré non assemblé dans l'organe de retrait ;
- la figure 9 est une vue en coupe longitudinale en perspective de l'organe de retrait avant assemblage sur un dispositif d'injection ;

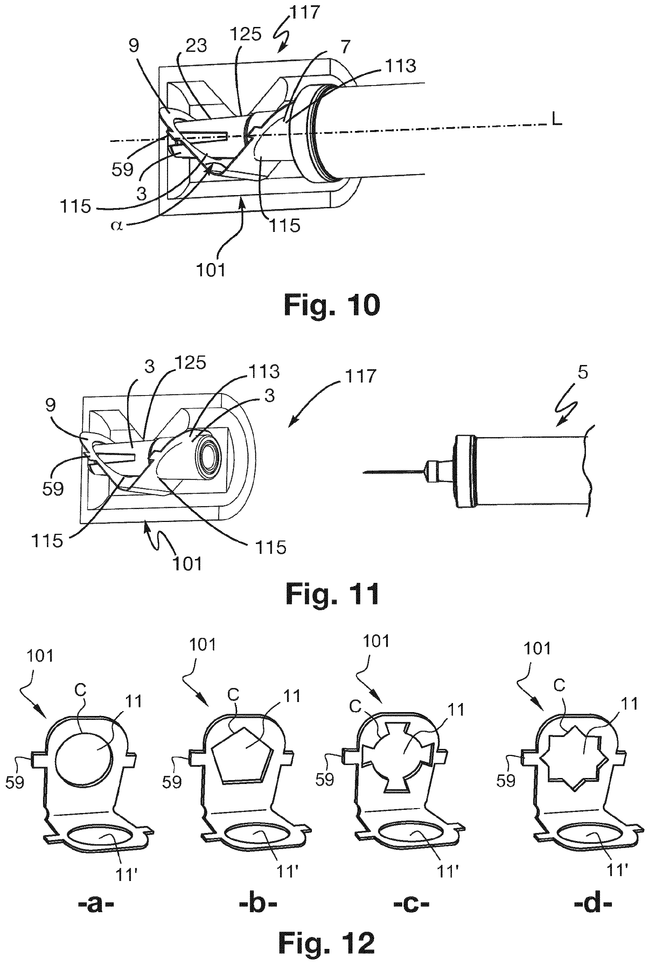

- la figure 10 est une vue en coupe longitudinale en perspective de l'organe de retrait assemblé sur un dispositif d'injection ;
- la figure 11 est une vue en coupe longitudinale en perspective de l'organe de retrait représenté après retrait du capuchon du dispositif d'injection ;
- les figures 12a à 12d représentent différentes variantes d'un élément de blocage d'un organe de retrait tel que représenté aux figures 8 à 11.

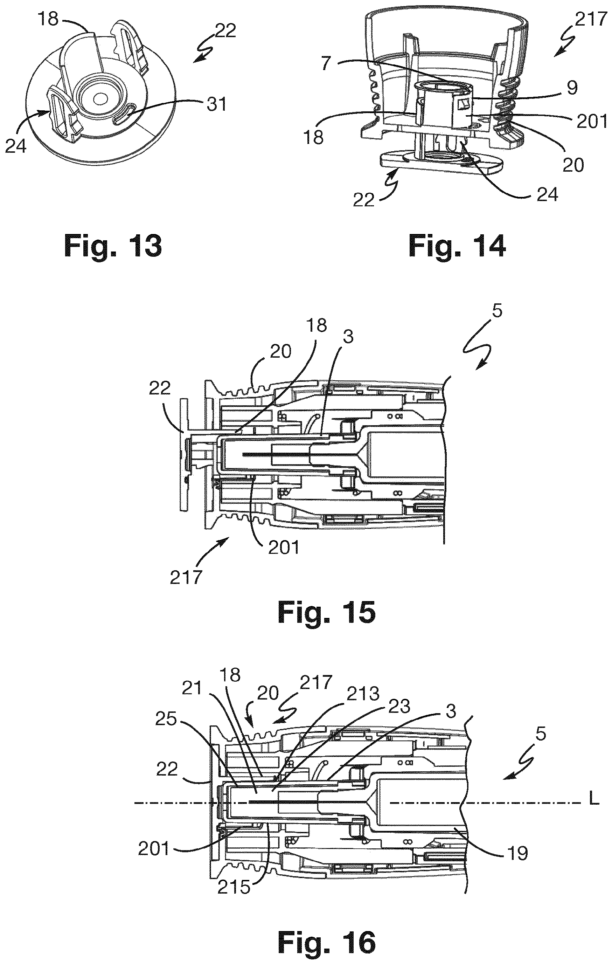

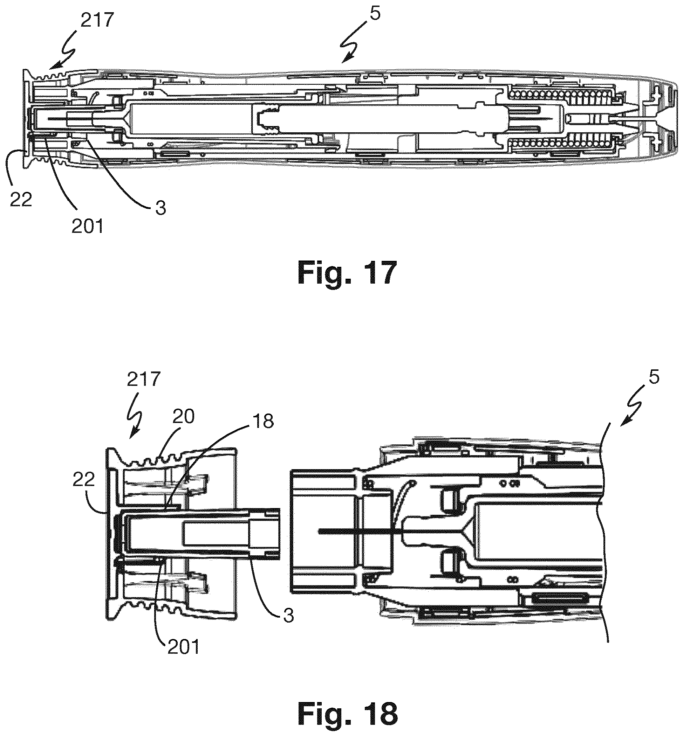

Un premier mode de réalisation du second principe de fonctionnement de l'invention est illustré par les figures 13 à 18 dans lesquelles :
- la figure 13 est une vue en perspective de haut d'un élément de poussée de l'organe de retrait ;
- la figure 14 est une vue en coupe en perspective d'un organe de retrait avec l'élément de poussée en position non enfoncée ;
- les figures 15 à 18 sont des vues en coupe longitudinale de l'organe de retrait et du dispositif d'injection dans lesquelles :
- en figure 15, l'organe de retrait est pré-assemblé sur le dispositif d'injection ;
- en figures 16 et 17, l'organe de retrait est assemblé au dispositif d'injection et en configuration de blocage, la figure 16 étant une vue agrandie partielle de la figure 17 ;
- en figure 18, l'organe de retrait et le capuchon sont retirés du dispositif d'injection.

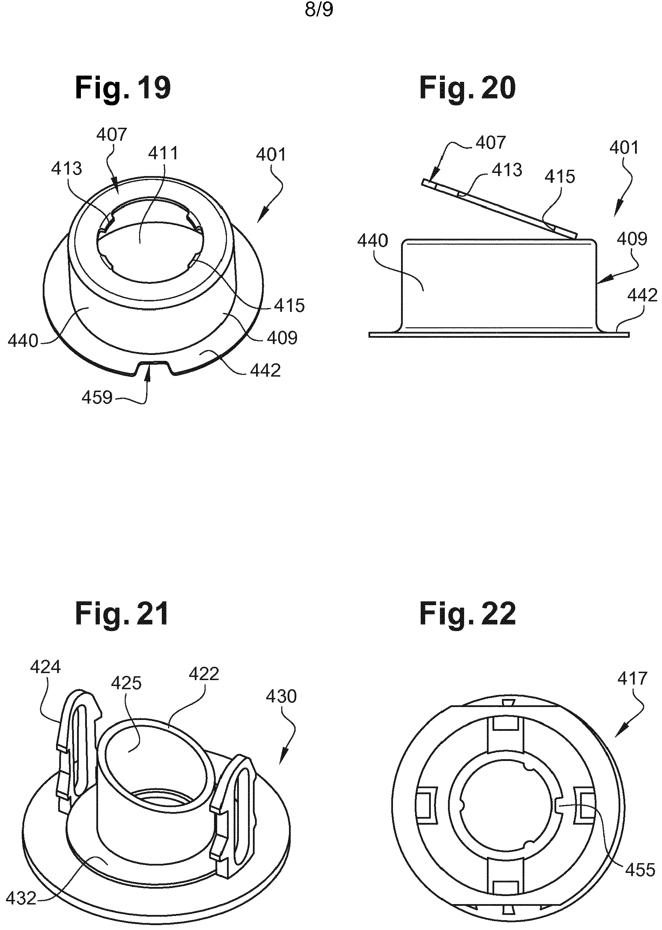

Un second mode de réalisation du second principe de fonctionnement de l'invention est illustré par les figures 19 à 23 dans lesquelles :
- la figure 19 est une vue en perspective de haut de l'élément de blocage ;
- la figure 20 est une vue de côté de l'élément de blocage ;
- la figure 21 est une vue en perspective de haut d'une extrémité mobile de l'organe de retrait ;
- la figure 22 est une vue en perspective de dessous d'un organe de retrait non assemblé avec l'extrémité mobile et l'élément de blocage;

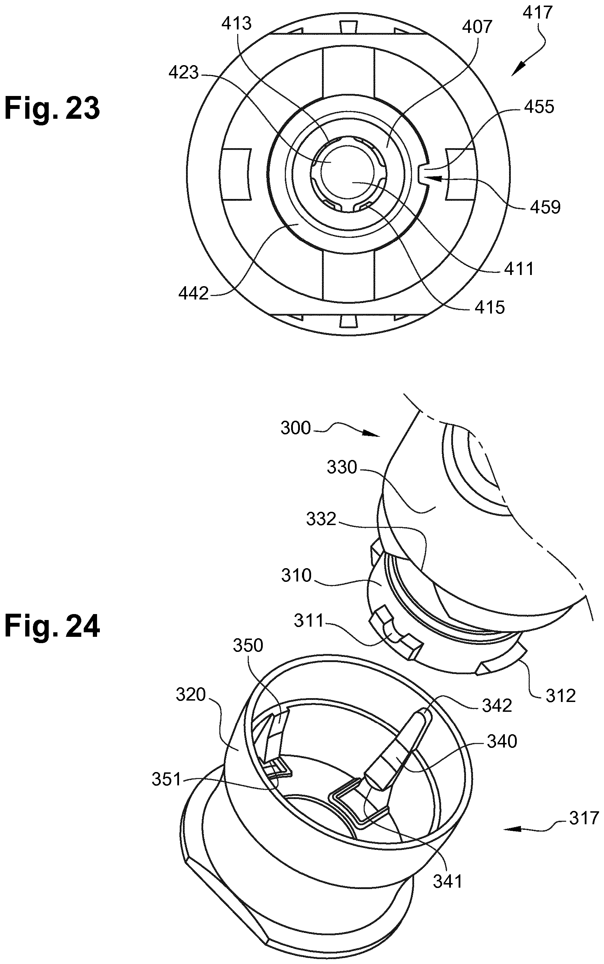

- la figure 23 est une vue en perspective de dessous de l'organe de retrait dans lequel l'élément de blocage est assemblé.

Deux modes de réalisations particuliers, compatibles avec les deux principes de fonctionnement, sont illustrés en figure 24, sur laquelle l'ensemble de l'organe de retrait et une partie d'un dispositif d'injection sont représentés en perspective.

En référence aux figures 1a-c, 2a-c, 3a-c l'organe de retrait 17, 117, 217 selon l'invention comporte un logement 23 apte à recevoir un capuchon 3 de protection d'aiguille. Le logement 23 présente un axe longitudinal L. Le logement 23 est délimité par une première 13, 113, 213, une seconde 15, 115, 215 et une troisième zone 25, 125, 225 d'appui du capuchon 3 destinées à servir d'appui au capuchon 3 et à le bloquer dans l'organe de retrait 17, 117, 217. L'organe de retrait 17, 117, 217 comporte un élément de blocage 1, 101, 201 portant la première zone d'appui 13, 113, 213 et mobile par rapport au logement 23 entre :
- une configuration d'insertion du capuchon 3 (figures 1b, 2a et traits pleins des figures 3a-c) dans laquelle des projections orthogonales de la première zone 13, 113, 213 et de la seconde zone 15, 115, 215 dans un plan transversal sensiblement perpendiculaire à l'axe longitudinal L sont séparées d'une distance d'insertion Di autorisant l'insertion du capuchon 3 dans l'organe de retrait 1, 101, 201, et
- une configuration de blocage (figures 1c, 2c et traits mixtes-fins des figures 3a-c) dans laquelle la première zone 13, 113, 213 et la seconde zone 15, 115, 215 sont situées dans des plans transversaux distincts, et dans laquelle les projections orthogonales de la première zone 13, 113, 213 et de la seconde zone 15, 115, 215 dans ledit plan transversal sont séparées d'une distance de blocage Db inférieure à la distance d'insertion Di et apte à bloquer la translation du capuchon 3 par rapport à l'organe de retrait 17, 117, 217 dans un sens de retrait opposé au sens d'insertion, dans laquelle le retrait du capuchon 3 par rapport à l'organe de retrait 1, 101, 201 est empêché.

La troisième zone 25, 125, 225 est destinée à empêcher le pivotement du capuchon 3 par rapport à l'une au moins des première 13, 113, 213 et seconde 15, 115, 215 zones dans un plan longitudinal, passant par l'axe longitudinal, au moins lors du passage de l'élément de blocage 1, 101, 201 entre ses configurations d'insertion et de blocage. Les première 13, 113, 213, seconde 15, 115, 215 et troisième zones 25, 125, 225 sont disposées en quinconce de part et d'autre de l'axe longitudinal L et dans au moins deux plans transversaux distincts lorsque l'élément de blocage 1, 101, 201 est en configuration de blocage.

Dans la configuration de blocage, le capuchon 3 est bloqué par arc-boutement entre les première zone 13, 113, 213, seconde zone 15, 115, 215 et troisième zone 25, 125, 225. Dans les modes de réalisation illustrés, l'élément de blocage 1, 101, 201 se présente sous la forme d'une plaque pourvue d'une ouverture de passage 11 définissant au moins la première zone 13, 113, 213. L'élément de blocage 1, 101, 201 peut se présenter sous toute autre forme adaptée.

L'organe de retrait 17, 117, 217 comporte au moins une butée axiale 14, 114, 214 limitant le déplacement de l'élément de blocage 1, 101, 201 par rapport à l'organe de retrait 17, 117, 217, et apte à bloquer la translation de l'élément de blocage 1, 101, 201 par rapport à l'organe de retrait 17, 117, 217 dans le sens de retrait du capuchon 3 dans l'organe de retrait 17, 117, 217. Cette butée axiale 14, 114, 214 peut être formée par un épaulement 14, 114, 214 contre lequel l'extrémité 30, 130, 230 de l'élément de blocage 1, 101, 201, opposée à une liaison pivot élastique P1, P3 (figures 3a et 3b), ou à un encastrement élastique P2 de celui-ci (figure 3c), vient en appui. La butée axiale 14, 114, 214 et l'extrémité 30, 130, 230 comportent des formes complémentaires La butée axiale peut être également formée par un encastrement de l'élément de blocage 1, 101, 201 dans l'organe de retrait 17, 117, 217, une liaison pivot ou tout autre moyen adapté.

Selon un premier principe de fonctionnement de l'invention, et en référence aux figures 1a-c, 3a, 3b, 3c, l'élément de blocage 1, 101 est déformable élastiquement entre la configuration d'insertion du capuchon 3 et la configuration de blocage du capuchon 3. La déformation élastique peut être assurée par l'intermédiaire d'une liaison pivot élastique P1 de l'élément de blocage 101 (figure 3b), d'un encastrement élastique P2 de l'élément de blocage 1 (figure 3c) ou par l'élasticité de l'élément de blocage lui-même (figures 9 à 12d). Selon ce premier principe de fonctionnement, on choisit le capuchon 3 et l'élément de blocage 1, 101 de sorte que :
a) Dans la configuration de repos de l'élément de blocage 1, 101 illustrée par les figures 1a :
   - l'élément de blocage 1, 101 forme un angle de repos ar avec l'axe longitudinal L ;
   - la distance de repos Dr qui sépare les projections orthogonales de la première zone 13, 113 et de la seconde zone 15,115 est inférieure au diamètre Dc du capuchon 3 dans la partie du capuchon 3 disposée à la hauteur des première zone 13, 113 et la seconde zone 15,115.
   Dans cette configuration de repos, le capuchon 3 ne peut passer au travers de l'ouverture de passage 11, ce qui est schématisé par la flèche barrée sur la figure 1a. La pression du capuchon 3 sur l'élément de blocage 1, 101 déforme élastiquement l'élément de blocage 1, 101 vers sa configuration d'insertion.
b) Dans la configuration d'insertion du capuchon 3 dans l'élément de blocage 1, 101 illustrée par les figures 1b :
   - l'élément de blocage 1, 101 forme un angle d'insertion ai avec l'axe longitudinal L, l'angle d'insertion ai étant inférieur à l'angle de repos ar ;
   - la distance d'insertion Di qui sépare les projections orthogonales de la première zone 13, 113 et de la seconde zone 15,115 est supérieure à la distance de repos Dr et supérieure au diamètre Dc du capuchon 3 dans la partie du capuchon 3 disposée à la hauteur des première zone 13, 113 et la seconde zone 15,115.
   Dans cette configuration d'insertion, le capuchon 3 peut passer au travers de l'ouverture de passage 11, ce qui est schématisé par la flèche non barrée sur la figure 1b. L'élément de blocage 1, 101 tend à se déformer élastiquement vers sa configuration de blocage.
c) Dans la configuration de blocage du capuchon 3 dans l'élément de blocage 1, 101 illustrée par les figures 1c :
   - l'élément de blocage 1, 101 forme un angle de blocage ab avec l'axe longitudinal L, l'angle de blocage ab étant supérieur à l'angle d'insertion ai et inférieur à l'angle de repos ar ;
   - la distance de blocage Db qui sépare les projections orthogonales de la première zone 13, 113 et de la seconde zone 15,115 est supérieure à la distance de repos Dr, inférieure à la distance d'insertion Di et est égale au diamètre Dc du capuchon 3 dans la partie du capuchon 3 disposée à la hauteur des première zone 13, 113 et la seconde zone 15,115, en tendant, de par l'élasticité de l'élément de blocage 1, 101, à être inférieure à ce diamètre Dc du capuchon 3.
   Dans cette configuration de blocage, le capuchon 3 est bloqué dans l'ouverture de passage 11, il ne peut progresser plus avant dans l'ouverture de passage, soit du fait de son appui dans le fond de l'organe de retrait, soit par l'appui du dispositif d'injection 5 sur l'organe de retrait. Le capuchon 3 ne peut pas non plus revenir en arrière, du fait de son maintien dans l'élément de blocage 1, 101 et du maintien de l'élément de blocage 1, 101 dans le capuchon 3. Ce double blocage est schématisé par la double flèche barrée sur la figure 1c. L'élément de blocage 1, 101 tend à se déformer élastiquement vers sa configuration de repos, maintenant ainsi le blocage du capuchon dans l'ouverture de passage 11.

Selon un second principe de fonctionnement de l'invention, et en référence aux figures 2a-c et 3a, l'élément de blocage 201 est déplacé et/ou déformé autour d'un point de pivotement Pi au moyen d'un élément de poussée 22 entre une configuration d'insertion (figure 2a) et une configuration de blocage (figure 2c), en passant par une configuration intermédiaire (figure 2b).

Selon ce second principe de fonctionnement, on choisit le capuchon 3 et l'élément de blocage 201 de sorte que :
a) Dans la configuration d'insertion du capuchon 3 dans l'élément de blocage 201 illustrée par les figures 2a :
   - l'élément de blocage 201 forme un angle d'insertion ai avec l'axe longitudinal L ;
   - la distance d'insertion Di qui sépare les projections orthogonales de la première zone 213 et de la seconde zone 215 est supérieure au diamètre Dc du capuchon 3 dans la partie du capuchon 3 disposée à la hauteur des première zone 213 et la seconde zone 215.
   Dans cette configuration d'insertion, le capuchon 3 peut passer au travers de l'ouverture de passage 11, ce qui est schématisé par la flèche non barrée sur la figure 2a.
   Une fois le capuchon 3 inséré dans l'ouverture de passage 11, l'élément de blocage 201 peut être déformé par l'élément de poussée 22 déplacé longitudinalement. Ce déplacement est symbolisé par la flèche représentée sur la figure 2b jusqu'à ce que l'élément de blocage 201 soit dans sa configuration de blocage.
b) Dans la configuration de blocage du capuchon 3 dans l'élément de blocage 201 illustrée par les figures 2c :
   - l'élément de blocage 201 forme un angle de blocage ab avec l'axe longitudinal L, l'angle de blocage ab étant supérieur à l'angle d'insertion ai et à un angle intermédiaire aint formé par l'élément de blocage 201 entre ses configurations de repos et de blocage ;
   - la distance de repos Dr qui sépare les projections orthogonales de la première zone 213 et de la seconde zone 215 est inférieure à la distance d'insertion Di et est égale au diamètre Dc du capuchon 3 dans la partie du capuchon 3 disposée à la hauteur des première zone 213 et seconde zone 215.
   Dans cette configuration de blocage, le capuchon 3 est bloqué dans l'ouverture de passage 11, il ne peut progresser plus avant dans l'ouverture de passage ni revenir en arrière, ce qui est schématisé par la double flèche barrée sur la figure 1c. L'élément de poussée 22 déplace l'élément de blocage 201 jusqu'à le déformer élastiquement ou de façon permanente de façon à garantir le maintien du capuchon 3.

Comme visible sur les figures 3a-c, la troisième zone 25, 125, 225 est destinée à servir d'appui au capuchon 3 pour empêcher son pivotement par rapport à l'une au moins des première zone 13, 113, 213 et seconde zone 15, 115, 215 dans un plan longitudinal. Les première 13, 113, 213, seconde 15, 115, 215 et troisième zones, 25, 125, 225 sont disposées en quinconce de part et d'autre de l'axe longitudinal L. Les première 13, 113, 213, seconde 15, 115, 215 et troisième zones 25, 125, 225 sont dans des plans transversaux distincts lorsque l'élément de blocage 1, 101, 201 est en configuration de blocage. La troisième zone 25, 125, 225 forme un moyen de guidage axial du capuchon 3 entre la configuration d'insertion et la configuration de blocage.

Selon un premier principe de fonctionnement et un premier mode de réalisation, comme représenté sur les figures 4a à 7 et 1a-c, l'organe de retrait 17 comporte un élément de blocage 1 se présentant sous forme d'une plaque pliée de façon à définir deux portions 7 et 9 s'étendant de part et d'autre d'un rayon de pliage définissant un axe de pivotement Pi des portions 7 et 9 l'une par rapport à l'autre. L'élément de blocage 1 et l'organe de retrait 17 comportent des formes complémentaires 29, 31 définissant une butée axiale apte à bloquer la translation de l'élément de blocage 1 par rapport à l'organe de retrait 17 dans le sens de retrait du capuchon 3 dans l'organe de retrait 17. Les deux portions 7 et 9 forment un angle α entre elles, un angle de repos ar voisin de 135° en configuration de repos, un angle d'insertion ai de 120° en configuration d'insertion, et un angle de blocage ab de 135° en configuration de blocage, en étant toutefois légèrement inférieur à l'angle de repos ar. Comme représenté, la portion 7 comprend une ouverture de passage 11 du capuchon 3. Dans ce mode de réalisation, l'élément de blocage 1 comprend une plaque pliée définissant les deux portions 7 et 9. Comme décrit plus loin, l'élément de blocage 1 peut être déformé de manière élastique ou permanente, par exemple autour d'un axe de pivotement Pi. Le contour C de l'ouverture de passage 11 définit une première zone d'appui 13 du capuchon 3, que l'on appelle «première zone » 13. Il définit également une seconde zone d'appui 15 du capuchon 3, que l'on appelle « seconde zone » 15. Dans ce mode de réalisation, la première zone 13 et la seconde zone 15 sont portées par l'élément de blocage 1. Dans ce mode de réalisation, la troisième zone non représentée est formée par l'appui du dispositif d'injection 5 sur l'organe de retrait 17.

Le logement 23 présente un axe longitudinal L, confondu avec l'axe longitudinal L du capuchon 3 lorsque celui-ci est dans le logement 23. Les première et seconde zones 13 et 15 sont disposées de part et d'autre de l'axe longitudinal L et situées dans des plans transversaux distincts lorsque l'élément de blocage 1 est en configuration de blocage, ce qui est visible sur la figure 7, et sur la vue supérieure de la figure 1c.

Dans un autre mode de réalisation non représenté, la seconde zone peut être portée par l'organe de retrait, la troisième zone peut être portée par l'élément de blocage.

L'élément de blocage 1 est mobile entre la configuration d'insertion du capuchon 3 (figures 1b et 6) et la configuration de blocage du capuchon 3 (figures 1c et 7). La portion 9 est ici une portion de fixation de l'élément de blocage 1 sur le reste d'un organe de retrait 17 (décrit plus loin) par le biais d'une liaison encastrement, plus précisément par encliquetage ou bouterollage. Ainsi, après le retrait du capuchon 3 par l'intermédiaire de l'élément de blocage 1, l'élément de blocage 1 est solidaire de l'organe de retrait 17, permettant ainsi de bloquer le capuchon 3 dans l'organe de retrait 17. L'élément de blocage 1 et une extrémité mobile 18' de l'organe de retrait 17 comportent des formes complémentaires 29 et 31 aptes à coopérer pour former une liaison de type encastrement. Celle-ci est apte à bloquer la translation de l'élément de blocage 1 par rapport à l'organe de retrait 17 dans un sens de retrait opposé au sens d'insertion du capuchon 3 dans l'organe de retrait 17.

Dans une variante de réalisation non représentée, l'élément de blocage comprend une plaque simple pourvue de l'ouverture de passage et dont un bord extérieur est solidarisé par une liaison de type encastrement sur le reste d'un organe de retrait. Dans cet autre mode de réalisation, le contour de l'ouverture opposé au bord de la plaque encastré forme la première zone d'appui.

Le dispositif d'injection 5 représenté en figures 6 et 7 comprend une seringue d'injection qui comprend un corps 19 pourvu d'une aiguille 21 creuse. Le dispositif d'injection 5 est destiné à administrer un produit par injection. L'organe de retrait 17 de ce mode de réalisation comprend un logement 23 pour le capuchon 3. Le logement 23 est délimité par au moins les première et seconde zones 13 et 15. Le logement 23 correspond à l'espace laissé libre pour que le capuchon 3 puisse être reçu dans l'organe de retrait 17, il a ici une forme générale cylindrique, munie de saillies et d'évidements.

Selon le premier principe de fonctionnement et un second mode de réalisation, comme représenté sur les figures 8 à 12d, l'organe de retrait 117 comporte un élément de blocage 101 se présentant sous forme d'une plaque pliée définissant les deux portions 7 et 9 sensiblement symétriques entre elles et déformables l'une par rapport à l'autre de manière élastique ou permanente. Les deux portions 7 et 9 s'étendent de part et d'autre d'un rayon de pliage définissant un axe de pivotement Pi. Les deux portions 7 et 9 forment un angle α entre elles, en configuration de repos un angle de repos ar voisin de 90°. La portion 7 est traversée par une ouverture de passage 11 définissant une première zone d'appui 113 du capuchon 3, que l'on appelle « première zone » 113, et une seconde zone d'appui 115 que l'on appelle « seconde zone ». Les première 113 et seconde 115 zones sont disposées de part et d'autre de l'axe longitudinal L du logement 23. La portion 9 est traversée par une ouverture de passage 11'. Dans ce mode de réalisation, la première zone 113 et la seconde zone 115 sont portées par l'élément de blocage 101.

L'organe de retrait 117 comporte un bossage faisant saillie de la paroi intérieure du logement 23 et définissant la troisième zone 125. Cette troisième zone 125 forme également un moyen de guidage axial du capuchon 3 entre la configuration d'insertion et la configuration de blocage.

L'élément de blocage 101 et l'organe de retrait 117 comportent des formes complémentaires: des rainures 55 de l'organe de retrait 117 qui s'arrêtent avant l'extrémité proximale de l'organe de retrait 117 et des ergots 59 sur l'élément de blocage 101. Les rainures 55 qui sont non débouchantes et les ergots 59 définissent une butée axiale apte à bloquer la translation de l'élément de blocage 101 par rapport à l'organe de retrait 117, dans le sens de retrait du capuchon 3 dans l'organe de retrait 117.

Ainsi, chacune des deux portions 7, 9 comprend respectivement une première ouverture de passage 11 et une seconde ouverture de passage 11' respectivement, ainsi que des ergots 59 coopérant avec deux rainures 55 du logement 23.

Dans ce mode de réalisation, la mobilité des première et seconde zones 113, 115 s'apparente à une liaison de type pivot mobile élastique tendant à ramener l'élément de blocage 101 dans sa configuration de blocage. Ainsi, l'élément de blocage 101 est déformable élastiquement. Le pivot est mobile en translation dans l'organe de retrait 117 grâce à la coopération entre les rainures 55 et les ergots 59.

L'organe de retrait 117 comprend une surface de préhension de l'organe de retrait 117 qui est une jupe externe de préhension de l'organe de retrait 117 par un utilisateur.

Le dispositif d'injection 5 comporte un dispositif de sécurité (non représenté) pour protéger l'utilisateur contre une piqûre de l'aiguille après utilisation, ou un dispositif auto-injecteur.

Comme illustré, le contour C de l'ouverture de passage 11 peut avoir une forme circulaire en figure 12a, la forme d'un pentagone en figure 12b, une forme circulaire munie de saillies intérieures en figure 12c, la forme d'une étoile à huit branches en figure 12d.

Pour le premier principe de fonctionnement, l'assemblage de l'organe de retrait 17, 117 sur le dispositif d'injection 5 comprend les étapes suivantes.

On insère le capuchon 3 au regard du logement 23, dans le sens d'insertion par exemple jusqu'à ce que celui-ci arrive en butée dans le fond de l'organe de retrait 17, 117. Le capuchon 3 est inséré de façon à exercer une pression sur l'élément de blocage 1, 101 pour le faire passer d'une configuration de repos vers une configuration d'insertion et augmenter la distance séparant les projections orthogonales jusqu'à la distance d'insertion Di. On stoppe l'insertion du capuchon 3 dans l'organe de retrait 17, 117. L'élément de blocage 1, 101 prend une configuration de blocage dans laquelle la distance séparant les projections orthogonales de la première zone 13, 113 et de la seconde zone 15, 115 dans un plan transversal, diminue pour atteindre une distance dite distance de blocage Db, et bloquer le capuchon 3 dans l'organe de retrait 17, 117.

Selon le second principe de fonctionnement, premier mode de réalisation, comme représenté sur les figures 13 à 18, l'organe de retrait 217 comporte un élément de poussée 22 déplaçable par rapport à une surface de préhension 20 de l'organe de retrait 217, lors de l'assemblage de l'organe de retrait 217 au dispositif d'injection 5, entre une position non enfoncée et une position enfoncée.

L'élément de poussée 22 comporte une patte de poussée 18 apte à solliciter l'élément de blocage 201 vers sa configuration de blocage du capuchon 3 lors du passage de l'élément de poussée 22 de sa position non enfoncée à sa position enfoncée. L'élément de poussée 22 comporte des moyens de fixation à l'organe de retrait 217, par exemple des pattes de fixation 24 (visibles sur la figure 13), aptes à se loger par déformation élastique dans une rainure prévue dans l'organe de retrait 217, les crans prévus sur les pattes de fixation 24 fiabilisant la fixation de l'élément de poussée 22 à l'organe de retrait 217 dans sa position enfoncée. L'élément de poussée 22 est donc pourvu de moyens de verrouillage 24 aptes à l'immobiliser par rapport au logement 23 dans la position enfoncée. Dans la position non enfoncée, l'élément de poussée 22 peut être pré-assemblé à l'organe de retrait 217 dans une position non enfoncée-pré-assemblée, sans toutefois que la patte de poussée 18 ne sollicite l'élément de blocage 201 vers sa configuration de blocage. A cet effet, les pattes de fixation 24 peuvent comporter des crans intermédiaires pour maintenir l'élément de poussée 22 dans cette configuration non enfoncée-pré-assemblée. L'organe de retrait 217 présente une extrémité pourvue de fenêtres traversées par la patte de poussée 18 et les pattes de fixation 24 de l'élément de poussée 22 en position non enfoncée-pré-assemblée et assemblée. L'élément de poussée 22 est ainsi en butée axiale contre l'organe de retrait 217.

Les figures 19 à 23 illustrent un second mode de réalisation du second principe de fonctionnement de l'invention, dans lequel l'organe de retrait 417 comprend une surface de préhension (non apparente sur les vues en perspective de dessous), une extrémité mobile 430 et un élément de blocage 401. L'élément de blocage 401 se présente sous la forme d'une plaque pliée de façon à définir deux portions 407 et 409 s'étendant de part et d'autre d'un rayon de pliage définissant un axe de pivotement des portions 407 et 409 l'une par rapport à l'autre. La portion 407 est pourvue d'une ouverture de passage 411 définissant les première et deuxième zones d'appui 413 et 415 qui délimitent un logement 423 de l'organe de retrait 417 apte à recevoir le capuchon de protection d'aiguille. La portion 409 forme une jupe 440 présentant à son extrémité distale une collerette 442. L'extrémité mobile 430 comporte des pattes de fixation 424 à l'organe de retrait 417, un cylindre tronqué qui constitue un élément de poussée 422 mobile par rapport à la surface de préhension de l'organe de retrait 417, et une embase 432. Le cylindre tronqué 422 s'élève sensiblement au centre de l'embase 432. Le cylindre tronqué 422, l'élément de blocage 401 et le capuchon 3 sont destinés à être placés de façon à présenter le même axe longitudinal (L) que le logement 423. Les pattes de fixation 424 de l'extrémité mobile 430 constituent des moyens de verrouillage de l'élément de poussée 422 qui sont aptes à l'immobiliser par rapport au logement 423 dans la position enfoncée. L'embase 432 forme un siège pour la collerette 442 de l'élément de blocage 401. La collerette 442 de l'élément de blocage 401 a un diamètre intérieur supérieur au diamètre extérieur du cylindre tronqué 422 de l'extrémité mobile 430. L'embase 432 comporte une surface plane définissant une butée axiale 432 pour la surface distale de la collerette 442, qui est également une surface plane. La butée axiale 432 est apte à bloquer la translation de l'élément de blocage 401 par rapport à l'organe de retrait 417 dans le sens de retrait du capuchon dans l'organe de retrait 417. Dans ce mode de réalisation, les points d'appui sont répartis sur la collerette 442 ce qui améliore le blocage de l'élément de blocage 401 dans l'organe de retrait 417. Ceci fiabilise l'organe de retrait.

Par ailleurs, la collerette 442 de l'élément de blocage 401 comporte une encoche 459 et l'organe de retrait 417 comporte un bossage 455. L'encoche 459 de l'élément de blocage 401 et le bossage 455 de l'organe de retrait 417 sont deux formes complémentaires qui définissent une butée angulaire 455 apte à bloquer la rotation de l'élément de blocage 401 par rapport à l'organe de retrait 417, de sorte que l'orientation angulaire de l'élément de blocage 401 par rapport à l'élément de poussée 422 permette le passage de la configuration d'insertion du capuchon 3 à la configuration de blocage du capuchon 3 sous l'action de l'élément de poussée 422. Dans un autre mode de réalisation non représenté, le bossage est porté par la collerette et l'encoche est portée par l'organe de retrait.

L'élément de poussée 422 porte la troisième zone d'appui 425. Celle-ci est destinée à servir d'appui au capuchon pour empêcher son pivotement par rapport à la première 413 et la seconde zones 415 dans un plan longitudinal, au moins lors du passage de l'élément de blocage entre ses configurations d'insertion et de blocage. Les première 413, seconde 415 et troisième 425 zones sont disposées en quinconce de part et d'autre de l'axe longitudinal et dans au moins deux plans transversaux distincts lorsque l'élément de blocage 401 est en configuration de blocage. Les trois zones d'appui 413, 415 et 425 délimitent le logement 423 pour le capuchon de protection d'aiguille non représenté ici. En configuration de blocage, la première zone 413 et la seconde zone 415 sont situées dans des plans transversaux distincts, d'avantage éloignés l'un de l'autre qu'en configuration d'insertion. Le passage présenté au capuchon lors de son insertion est plus large que le passage présenté au capuchon en configuration de blocage, en assurant ainsi le blocage du capuchon dans l'organe de retrait 417 par un arc-boutement de l'élément de blocage 401.

Pour le second principe de fonctionnement, l'assemblage de l'organe de retrait 217, 417 sur le dispositif d'injection 5 comprend les étapes suivantes.

On insère le capuchon 3 au regard du logement 23, 423, dans le sens d'insertion.

On presse dans la direction longitudinale l'élément de poussée 22, 422 par rapport à la surface de préhension 20 de l'organe de retrait 17, 417, entre une position non enfoncée et une position enfoncée dans laquelle l'élément de blocage 201, 401 est en configuration de blocage du capuchon 3 sous l'action de l'élément de poussée 22, 422.

Dans le deuxième mode de réalisation du second principe de fonctionnement, préalablement à l'insertion du capuchon 3, les formes complémentaires 459, 455 de l'élément de blocage 401 et de l'organe de retrait 417 sont positionnées de manière à bloquer en rotation l'élément de blocage 401 par rapport à l'organe de retrait 417.

Dans un mode de réalisation particulier réalisé selon le premier ou le second principe de fonctionnement, et représenté en figure 24, l'organe de retrait est destiné à équiper un dispositif d'injection comprenant un dispositif auto-injecteur. Le dispositif auto-injecteur 300 comprend un manchon 310 d'extrémité, destiné à être en contact avec la peau d'un utilisateur au cours de l'injection. Le manchon 310 d'extrémité, qui forme l'extrémité distale du dispositif d'injection, est rapporté sur l'extrémité distale d'un support de seringue (non représenté), lui-même configuré pour loger une seringue d'injection. Il est également équipé d'un boîtier extérieur 330, dont l'extrémité distale est ici représentée. Dans ce mode de réalisation, l'organe de retrait 317 comporte une surface de préhension 320. L'organe de retrait 317 comporte en outre deux nervures 340 faisant saillie de la paroi intérieure de l'organe de retrait 317. Ces nervures 340 de l'organe de retrait 317 et le manchon 310 d'extrémité comportent des formes complémentaires 341 et 311 aptes à coopérer pour bloquer la rotation de l'organe de retrait 317 par rapport au manchon 310. L'organe de retrait 317 comporte également des nervures 350 similaires aux nervures 340 mais ne comportant pas de forme 341. Par conséquent ces nervures 350 sont plus courtes que les nervures 340 et présentent une extrémité plane 351. Ces extrémités planes 351 coopèrent avec des butées planes 312 du manchon 310 d'extrémité, de manière à bloquer en translation l'organe de retrait 317 sur l'extrémité distale 310 du dispositif d'injection. De plus, les extrémités planes 351 et extrémités planes 351 butées planes 312 sont positionnées respectivement autour de l'organe de retrait 317 et du manchon 310 de manière à ce qu'une rotation de l'organe de retrait 317 par rapport au manchon 310 désengage les butées planes 312 des extrémités plane 351 et permette ainsi le retrait axial de l'organe de retrait 317 de l'extrémité distale 310. Les formes 341 et 311 sont situées respectivement à l'extrémité distale de la nervure 340 et à l'extrémité distale du manchon 310 d'extrémité. Dans l'exemple représenté en figure 24, les formes 341 et 311 sont des arcs de cercle. Ces formes 341 et 311 peuvent cependant prendre toute forme géométrique permettant un blocage en rotation. Grâce à la coopération entre ces deux formes 341 et 311, l'organe de retrait 317 est positionné angulairement sur le manchon 310 d'extrémité, et retenu dans cette position. De ce fait, le retrait de l'organe de retrait 317, permettant le retrait du capuchon de protection (non représenté ici), nécessite une rotation d'un certain angle de l'organe de retrait 317 par rapport au manchon 310 d'extrémité, ce qui permet d'exercer un contrôle sur le retrait de l'organe de retrait, et donc du capuchon de protection. De ce fait, en cas de chute du dispositif auto-injecteur, la chute de l'organe de retrait, c'est-à-dire la séparation entre l'organe de retrait et le dispositif auto-injecteur, est empêchée, de même que l'activation involontaire du dispositif auto-injecteur. Les extrémités planes 351 des nervures 350 et les butées planes 312 du manchon 310 d'extrémité renforcent le contrôle exercé sur la chute de l'organe de retrait.

Ce mode de réalisation particulier peut être complété par d'autres caractéristiques, également représentées en figure 24. Dans cette variante, les nervures 340 de l'organe de retrait 317 et le boîtier extérieur 330 comportent des éléments 342 et 332 destinés à coopérer. Ces éléments 342 et 332 sont situés respectivement à l'extrémité proximale de la nervure 340 et à l'extrémité distale du boîtier extérieur 330. Dans l'exemple représenté en figure 19, l'élément 342 est un arc de cercle et l'élément 332 est un évidement en forme de trapèze. Ainsi, l'élément 342 peut coopérer avec les pentes de l'élément 332 du boîtier extérieur 330, ce qui permet une translation par rotation de l'organe de retrait 317 sur le boîtier extérieur 330. Plus précisément, lorsque l'organe de retrait 317 est assemblé sur le dispositif auto-injecteur 300, la forme 342 de la nervure 340 est positionnée contre la partie la plus proximale de la forme 332 du boîtier extérieur 330. La pente de la forme 332 facilite la translation de l'organe de retrait par rotation et la libération de la forme 342. Ceci facilite donc le retrait de l'organe de retrait 317 du dispositif d'injection, et donc le retrait du capuchon de protection.

L'invention n'est pas limitée aux modes de réalisation présentés et d'autres modes de réalisation apparaîtront clairement à l'homme du métier. Il est notamment possible d'utiliser l'organe de retrait pour un dispositif d'injection distinct d'une seringue d'injection, par exemple une cartouche sur laquelle est rapportée une aiguille. Le dispositif d'injection peut également comprendre un dispositif de sécurité ou un dispositif auto-injecteur.

## Revendications

1. Organe de retrait (17, 117, 217, 317, 417) d'un capuchon (3) de protection d'aiguille, pour un dispositif d'injection (5) comprenant une aiguille (21) et un capuchon (3) amovible de protection d'aiguille (21), l'organe de retrait (17, 117, 217, 317, 417) comprenant un logement (23, 423) présentant un axe longitudinal (L) et étant apte à recevoir le capuchon (3) selon un sens d'insertion, le logement (23, 423) étant délimité par au moins une première zone d'appui (13, 113, 213, 413) et une seconde zone d'appui (15, 115, 215, 415) dites première et seconde zones destinées à servir d'appui au capuchon (3) logé dans le logement (23, 423) la première zone (13, 113, 213, 413) étant portée par un élément de blocage (1, 101, 201, 401) mobile par rapport au logement (23, 423) entre :
- au moins une configuration d'insertion du capuchon (3), dans laquelle des projections orthogonales de la première zone (13, 113, 213, 413) et de la seconde zone (15, 115, 215, 415) dans un plan transversal sensiblement perpendiculaire à l'axe longitudinal (L), lesdites projections orthogonales délimitant un passage présenté au capuchon (3) lors de son insertion dans un sens d'insertion, et lesdites projections étant séparées d'une distance d'insertion (Di), et
- une configuration de blocage du capuchon (3), dans laquelle la première zone (13, 113, 213, 413) et la seconde zone (15, 115, 215, 415) sont situées dans des plans transversaux distincts davantage éloignés l'un de l'autre qu'en configuration d'insertion, et dans laquelle les projections orthogonales de la première zone (13, 113, 213, 413) et de la seconde zone (15, 115, 215, 415) dans ledit plan transversal sont séparées d'une distance de blocage (Db) inférieure à la distance d'insertion (Di) et apte à bloquer la translation du capuchon (3) par rapport à l'organe de retrait (17, 117, 217, 317, 417) dans un sens de retrait opposé au sens d'insertion, si bien que le passage présenté au capuchon (3) lors de son insertion dans le sens d'insertion est plus large que le passage présenté au même capuchon (3) en configuration de blocage, en assurant ainsi le blocage du capuchon (3) dans l'organe de retrait (17, 117, 217, 317, 417) par un arc-boutement de l'élément de blocage (1, 101, 201, 401),
dans lequel le logement (23, 423) est délimité en outre par une troisième zone d'appui dite troisième zone (25, 125, 225, 425), destinée à servir d'appui au capuchon (3) pour empêcher son pivotement par rapport à l'une au moins desdites première (13, 113, 213, 413) et seconde (15, 115, 215, 415) zones dans un plan longitudinal, au moins lors du passage de l'élément de blocage (1, 101, 201, 401) entre ses configurations d'insertion et de blocage, les première (13, 113, 213, 413), seconde (15, 115, 215, 415) et troisième zones (25,125, 225, 425) étant disposées en quinconce de part et d'autre de l'axe longitudinal (L) et dans au moins deux plans transversaux distincts lorsque l'élément de blocage (1, 101, 201, 401) est en configuration de blocage.

2. Organe de retrait (17, 117, 217, 317, 417) selon la revendication précédente, dans lequel l'élément de blocage (1, 101, 201, 401) comprend une plaque délimitant au moins une première ouverture de passage (11, 411) du capuchon (3) dont le contour (C) définit au moins la première zone (13, 113, 213, 413), de préférence le contour (C) de la première ouverture de passage (11) définit également au moins l'une des seconde zone (15, 115, 215, 415) et troisième zone.

3. Organe de retrait (17, 117, 217, 417) selon l'une quelconque des revendications précédentes, dans lequel l'élément de blocage (1, 101, 201, 401) et l'organe de retrait (17, 117, 217, 417) comportent des formes complémentaires (14, 114, 214, 30, 130, 230, 29, 31, 55, 59) ou chacun une surface plane (432, 442) définissant une butée axiale (14, 114, 214, 432) apte à bloquer la translation de l'élément de blocage (1, 101, 201, 401) par rapport à l'organe de retrait (17, 117, 217, 417) dans le sens de retrait du capuchon (3) dans l'organe de retrait (17, 117, 217, 417).

4. Organe de retrait (17, 117, 217) selon l'une quelconque des revendications précédentes, dans lequel l'élément de blocage (1, 101, 201) est mobile par rapport au logement (23) de façon à prendre également une configuration de repos, dans laquelle les projections orthogonales de la première zone (13, 113, 213) et de la seconde zone (15, 115, 215) dans un plan transversal sont séparées d'une distance de repos (Dr) inférieure à la distance de blocage (Db).

5. Organe de retrait (17, 117, 217, 417) selon l'une quelconque des revendications précédentes, dans lequel la première zone (13, 113, 213, 413) et la seconde zone (15, 115, 215, 415) sont portées par l'élément de blocage (1, 101, 201, 401).

6. Organe de retrait (17, 117, 217, 417) selon la revendication précédente, dans lequel l'élément de blocage (1, 101, 201, 401) est une plaque pliée de façon à définir deux portions (7, 9, 407, 409) s'étendant de part et d'autre d'un axe de pivotement (Pi) des portions (7, 9, 407, 409) l'une par rapport à l'autre, au moins une des deux portions (7, 9, 407, 409) comprenant une ouverture de passage (11, 411) du capuchon (3), les deux portions (7, 9, 407, 409) formant un angle (α) entre elles.

7. Organe de retrait (17, 117, 217, 417) selon l'une quelconque des revendications précédentes, dans lequel l'élément de blocage (1, 101, 201, 401) est déformable élastiquement entre au moins la configuration d'insertion du capuchon (3) et la configuration de blocage du capuchon (3).

8. Organe de retrait (217, 417) selon l'une quelconque des revendications précédentes, dans lequel l'élément de blocage (201, 401) passe de la configuration d'insertion du capuchon (3) à la configuration de blocage du capuchon (3) sous l'action d'un élément de poussée (22, 422) mobile par rapport au logement (23, 423), l'élément de poussée (22, 422) étant distinct du capuchon (3), entre une position non enfoncée dans laquelle l'élément de blocage (201, 401) est dans la configuration d'insertion et une position enfoncée dans laquelle l'élément de blocage (201, 401) est en configuration de blocage du capuchon (3) sous l'action de l'élément de poussée (22, 422), l'élément de poussée (22, 422) étant pourvu de moyens de verrouillage (24, 424) aptes à l'immobiliser par rapport au logement (23, 423) dans la position enfoncée.

9. Organe de retrait (317) selon l'une quelconque des revendications précédentes, comportant au moins une extrémité plane (351) complémentaire d'au moins une butée plane (312) d'une extrémité distale (310) du dispositif d'injection, l'extrémité plane (351) et la butée plane (312) étant aptes à coopérer ensemble de manière à bloquer en translation l'organe de retrait (317) sur l'extrémité distale (311) du dispositif d'injection.

10. Organe de retrait (317) selon la revendication précédente, dans lequel l'extrémité plane (351) et la butée plane (312) sont positionnées de manière à ce qu'une rotation de l'organe de retrait (317) par rapport au manchon (310) désengage la butée plane (312) de l'extrémité plane (351).

11. Organe de retrait (317) selon l'une quelconque des revendications précédentes, comportant une forme (341) complémentaire d'une forme (311) d'une extrémité distale (310) du dispositif d'injection, les deux forme (311, 341) étant aptes à coopérer ensemble de manière à bloquer en rotation l'organe de retrait (317) sur l'extrémité distale (311) du dispositif d'injection.

12. Dispositif d'injection (5) comprenant un organe de retrait (17, 117, 217) selon l'une quelconque des revendications précédentes, le dispositif d'injection (5) comportant de préférence un dispositif de sécurité pour protéger l'utilisateur contre une piqûre de l'aiguille (21) après utilisation.

13. Procédé d'assemblage d'un organe de retrait (17, 117, 217, 417) sur un dispositif d'injection (5) comprenant une aiguille (21) et un capuchon (3) amovible de protection d'aiguille (21), l'organe de retrait (17, 117, 217, 417) comprenant un logement (23, 423) présentant un axe longitudinal (L) et étant apte à recevoir le capuchon (3) selon un sens d'insertion, le logement (23, 423) étant délimité par au moins une première zone (13, 113, 213, 413) et seconde zone (15, 115, 215, 415) destinées à servir d'appui au capuchon (3) logé dans le logement (23, 423), la première zone (13, 113, 213, 413) étant portée par un élément de blocage (1, 101, 201, 401) mobile par rapport au logement (23, 423), le procédé comprenant au moins les étapes suivantes :
- on insère le capuchon (3) au regard du logement (23, 423), dans le sens d'insertion, l'élément de blocage (1, 101, 201, 401) étant dans une configuration d'insertion du capuchon (3), dans laquelle les projections orthogonales de la première zone (13, 113, 213, 413) et de la seconde zone (15, 115, 215, 415) dans un plan transversal sensiblement perpendiculaire à l'axe longitudinal (L) sont séparées d'une distance d'insertion (Di),
- on stoppe l'insertion du capuchon (3) dans l'organe de retrait (17, 117, 217, 417),
- l'élément de blocage (1, 101, 201, 401) prend une configuration de blocage du capuchon (3) dans laquelle la première zone (13, 113, 213, 413) et la seconde zone (15, 115, 215, 415) sont situées dans des plans transversaux distincts, les projections orthogonales de la première zone (13, 113, 213, 413) et de la seconde zone (15, 115, 215, 415) dans ledit plan transversal étant séparées d'une distance de blocage (Db), inférieure à la distance d'insertion (Di) et apte à bloquer la translation du capuchon (3) par rapport à l'organe de retrait (17, 117, 217, 417) dans le sens de retrait opposé au sens d'insertion du capuchon (3).

14. Procédé d'assemblage selon la revendication précédente, dans lequel on insère le capuchon (3) au regard du logement (23) de façon que le capuchon (3) exerce une pression sur l'élément de blocage (1, 101) pour le faire passer d'une configuration de repos dans laquelle les projections orthogonales de la première zone (13, 113) et de la seconde zone (15, 115) dans un plan transversal sont séparées d'une distance de repos (Dr) inférieure à la distance de blocage (Db) vers une configuration d'insertion et augmenter la distance séparant lesdites projections orthogonales jusqu'à la distance d'insertion (Di).

15. Procédé d'assemblage selon la revendication 13, dans lequel on fait passer l'élément de blocage (201, 401) de la configuration d'insertion du capuchon (3) à la configuration de blocage du capuchon (3) en déplaçant la première zone (213, 413) par rapport à la seconde zone (215, 415) au moyen d'un élément de poussée (22, 422), distinct du capuchon (3), que l'on déplace par rapport au logement (23, 423) entre une position non enfoncée dans laquelle l'élément de blocage (201, 401) est dans la configuration d'insertion et une position enfoncée dans laquelle l'élément de blocage (201, 401) est en configuration de blocage du capuchon (3), et on bloque l'élément de poussée (22, 422) par rapport au logement (23, 423) dans la position enfoncée.
